# EUROPÄISCHE PATENTSCHRIFT

(11) **EP 1 153 039 B1**
(45) Veröffentlichungstag und Bekanntmachung des Hinweises auf die Patenterteilung: **12.01.2005**
(21) Anmeldenummer: 00920437.1
(22) Anmeldetag: 18.02.2000
(51) Int. Cl.: C07K 16/28, A61K 39/395

(54) **VERWENDUNG VON BLOCKIERENDEN ANTI-TSH-REZEPTOR-ANTIKÖRPERN BEI DER THERAPIE VON HYPERTHYREOSEN SOWIE MONOKLONALE ANTIKÖRPER FÜR EINE SOLCHE VERWENDUNG**
USE OF BLOCKING ANTI-TSH-RECEPTOR-ANTIBODIES IN THE THERAPY OF HYPERTHYREOSES AND MONOCLONAL ANTIBODIES FOR A USE OF THIS TYPE
UTILISATION D'ANTICORPS DE BLOCAGE AGISSANT CONTRE LE RECEPTEUR DE TSH POUR TRAITER L'HYPERTHYROIDIE, ET ANTICORPS MONOCLONAUX FAISANT L'OBJET D'UNE TELLE UTILISATION

(30) Priorität: 19.02.1999 DE 19907094
(43) Veröffentlichungstag der Anmeldung: 14.11.2001
(73) Patentinhaber: B.R.A.H.M.S Aktiengesellschaft, 16761 Hennigsdorf (DE)
(72) Erfinder: BERGMANN, Andreas, 12351 Berlin (DE); STRUCK, Joachim, 12161 Berlin (DE); MORGENTHALER, Nils, Gernot, 12099 Berlin (DE); JOHNSTONE, Alan P., Cheam, Surrey SM3 8DE (GB); SHEPHERD, Philip S., Carshalton Beeches, Surrey SM5 4DP (GB)
(74) Vertreter: Andrae, Steffen, Dr.
(86) Internationale Anmeldenummer: PCT/EP2000/001304
(87) Internationale Veröffentlichungsnummer: WO 2000/049050

(56) Entgegenhaltungen:
- ORGIAZZI J ET AL: "[Thyroid-stimulating hormone receptor and thyroid diseases]. Recepteur de la thyreostimuline (TSH) et dysthyroidie." REVUE DU PRATICIEN, (1994 MAY 1) 44 (9) 1184-91. , XP000944211
- NICHOLSON, L. B. ET AL: "Monoclonal antibodies to the human TSH receptor: epitope mapping and binding to the native receptor on the basolateral plasma membrane of thyroid follicular cells" J. MOL. ENDOCRINOL. (1996), 16(2), 159-170 , XP000943987
- SEETHARAMAIAH, GATTADAHALLI S. ET AL: "Generation and characterization of monoclonal antibodies to the human thyrotropin (TSH) receptor: antibodies can bind to discrete conformationa or linear epitopes and block TSH binding" ENDOCRINOLOGY (1995), 136(7), 2817-24 , XP000944028
- SHEPHERD, P. S. ET AL: "Identification of an important thyrotrophin binding site on the human thyrotrophin receptor using monoclonal antibodies" MOL. CELL. ENDOCRINOL. (1999 MARCH 25), 149(1-2), 197-206 , XP000944024

## Beschreibung

Die vorliegende Erfindung betrifft die Verwendung von blokkierenden Anti-TSH-Rezeptor-Antikörpern im Rahmen der Therapie von Hyperthyreosen wie Morbus Basedow, d.h. zur Behandlung bzw. zur Herstellung von Arzneimitteln zur Behandlung derartiger Erkrankungen, sowie für eine solche Verwendung besonders gut geeignete monoklonale Antikörper.

Es ist bekannt, daß zahlreiche Erkrankungen, an denen die Schilddrüse beteiligt ist, Autoimmunerkrankungen sind, bei denen Autoantikörper gegen molekulare Strukturen der Schilddrüse gebildet werden, die im Zusammenhang mit der Erkrankung beginnen, als Autoantigene zu wirken. Die wichtigsten bekannten Auotantigene der Schilddrüse sind dabei Thyreoglobulin (Tg), die Schilddrüsenperoxidase (TPO) und insbesondere der TSH-Rezeptor (TSHr) (vgl. Furmaniak J et al., Autoimmunity 1990, Vol. 7, S. 63-80).

Der TSH-Rezeptor ist ein in der Schilddrüsenmembran lokalisierter Rezeptor, an den das von der Hypophyse ausgeschüttete Hormon TSH (Thyroid-stimulierendes Hormon oder Thyreotropin) bindet und dadurch die Ausschüttung der eigentlichen Schilddrüsenhormone, insbesondere des Thyroxins, auslöst. Der TSH-Rezeptor gehört zur Rezeptor-Familie der G-Proteingekoppelten Glykoprotein-Rezeptoren mit einer großen aminoterminalen extrazellulären Domäne, zu der auch der LH/CGund der FSH-Rezeptor gehören. Eine Aufklärung der chemischen Struktur des TSH-Rezeptors, d.h. der Sequenz der für ihn codierenden DNA sowie der daraus ableitbaren Aminosäuresequenz des Rezeptors selbst, gelang Ende 1989 (vgl. Libert F. et al., Biochem. Biophys. Res. Commun. 165: 1250-1255; Nagayama Y. et al., Biochem. Biophys. Res. Commun. 165: 1184-1190; vgl. auch EP-A-0433509 bzw. WO-A-91/09121; sowie WO-A-91/09137; WO-A-91/10735 und WO-A-91/03483; ferner Yuji Nagayama & Basil Rapoport, in: Molecular Endocrinology, Vol. 6 No. 2, S. 145-156 und die darin zitierte Literatur).

Wegen der Ausbildung von Autoantikörpern gegen der TSH-Rezeptor (auch generell abgekürzt TRAb) im Rahmen von verschiedenen Schilddrüsen-Autoimmunerkrankungen hat die Bestimmung von derartigen Autoantikörpern eine erhebliche klinische Bedeutung, insbesondere für die Diagnose des Morbus Basedow (Basedow Krankheit; englisch: Graves' disease).

Abgesehen von Bestimmungsverfahren, bei denen Versuchstiere oder spezielle Zellkulturen eine Rolle spielen und die heute vor allem von historischem Interesse sind (vgl. Schumm-Draeger et al., Akt. Endokr. Stoffw. 10 (1989), S. 90-102)), können TSH-Rezeptor-Autoantikörper bisher im wesentlichen nach zwei Verfahrensprinzipien bestimmt werden (vgl. Morgenthaler N.G. et al., Horm. Metab. Res. 30 (1998), S. 162-168) :

Bei Zellstimulationstests äußert sich die Anwesenheit von stimulierenden TSH-Rezeptor-Autoantikörpern, die in der Literatur häufig mit den Abkürzungen TSAb oder TSI (TSI = thyroid stimulating immunoglobulins) bezeichnet werden, dadurch, daß bestimmte Funktionen von geeigneten Zellen, die in ihrer Zellmembran natürliche oder rekombinante TSH-Rezeptoren aufweisen und mit TSH und/oder einer Autoantikörper enthaltenden Probe in Kontakt kommen, durch Stimulation ausgelöst oder vestärkt werden, insbesondere die Bildung von cAMP (cyclischem Adenosinmonophosphat). Blockierende TSH-Rezeptor-Antikörper (TBAb) können an der Abschwächung der Wirkung von TSH erkannt werden. Da derartige Bioassays in ihrer Durchführung sehr aufwendig sind, liegt ihre Bedeutung derzeit weniger auf dem Gebiet der klinischen Routinediagnostik als auf dem der Grundlagenforschung. Sie stellen ein wertvolles Hilfsmittel zur Aufklärung der genauen Art der Wechselwirkung zwischen Antikörpern und der funktionellen Feinstruktur des TSH-Rezeptors dar. So wird in der Veröffentlichung von N.G. Morgenthaler et al. in Horm. Metab. Res. 30 (1998), S. 162-168 beschrieben, wie bestimmte CHO-Zellen, die so transformiert wurden, daß sie den vollständigen rekombinaten TSH-Rezeptor exprimieren und die als JP09 CHO-Zellen bezeichnet werden (Perret J et al., Biochem Biophys Res Commun 1990; 171:1044-50), erfolgreich dazu verwendet werden können, in unfraktionierten Seren oder daraus durch Reinigung erhältlichen IgG-Fraktionen stimulierende (TSAb) und/oder blockierende Autoantikörper (TBAb) nachzuweisen und voneinander zu unterscheiden bzw. deren Auftreten und ihre relativen Anteile in einer biologischen Probe zu ermitteln. Auf die in der genannten Veröffentlichung beschriebene Methodik wird nachfolgend im experimentellen Teil Bezug genommen, und auf den gesamten Inhalt dieser Veröffentlichung sowie die darin als Literaturzitate genannten weiteren Veröffentlichungen wird zur Ergänzung der vorliegenden Beschreibung sowie als weiterführende vertiefende Literatur ausdrücklich Bezug genommen.

Alternativ dazu können TSH-Rezeptor-Autoantikörper (TRAb) auch unter Verwendung von kompetitiven Rezeptorbindungsassays, insbesondere Radiorezeptorassays, bestimmt werden, z.B. unter Verwendung des TRAK Assay® der B.R.A.H.M.S Diagnostica GmbH. Zur Bestimmung von TSH-Rezeptor-Autoantikörpern wird nach der herkömmlichen Variante dieses Verfahrens so vorgegangen, daß man die zu bestimmenden Autoantikörper aus einer Serumprobe in flüssiger Phase mit einem radioaktiv markierten bovinen TSH-Kompetitor um die Bindungsstellen eines Detergens-solubilisierten porcinen TSH-Rezeptors konkurrieren läßt (vgl. Southgate, K. et al., Clin. Endocrinol. (Oxford) 20, 539-541 (1984); Matsuba T. et al., J.Biochem.118, S.265-270 (1995); EP 719 858 A2; Produktinformation zum TRAK-Assay® der Firma B.R.A.H.M.S Diagnostica GmbH). Um das an die Rezeptor-Präparation gebundene markierte TSH zu bestimmen, wird nach Abschluß der Inkubation der TSH-Rezeptor mit einem Fällungsreagens und einem anschließenden Zentrifugierschritt von der flüssigen Phase abgetrennt. Die Bestimmung des Rezeptor-gebundenen markierten TSH erfolgt durch Messung der im Sediment gebundenen Radioaktivität. Da die Bestimmung auf einer Konkurrenz (Kompetition) zwischen markiertem TSH und den zu bestimmenden Autoantikörpern um gemeinsame Bindungsstellen auf dem TSH-Rezeptor beruht, werden bei diesem Verfahren alle solchen und nur solche Autoantikörper erfaßt, die tatsächlich mit TSH kompetieren. Solche kompetierenden, zur Inhibierung der TSH-Bindung befähigten Autoantikörper werden in der Literatur auch als TBII (TBII = thyrotropin-binding inhibitory immunoglobulin) bezeichnet, und das Ausmaß ihrer Aktivität wird auch als prozentuale sogenannte TBII-Aktivität angegeben.

Es ist nunmehr seit längerem bekannt, daß bei Autoimmunerkrankungen der Schilddrüse heterogene Autoantikörperpopulationen unterschiedlicher Zusammensetzung gebildet werden. Dabei sind die stimulierenden und die mit TSH kompetierenden Autoantikörper nur teilweise identisch, d.h. es gibt stimulierende Autoantikörper, die nicht mit TSH kompetieren, und es gibt auch mit TSH kompetierende Autoantikörper, die nicht stimulierend wirken. Die Heterogenität der Autoantikörperpopulationen führte dazu, daß mit den früheren Radiorezeptorassays zum Nachweis von Autoantikörpern gegen den TSH-Rezeptor derartige Autoantikörper nur bei 80-90% der Morbus Basedow Patienten nachweisbar waren. Erst mit den moderneren Rezeptorbindungsassays der sog. "zweiten Generation", bei denen anstelle porciner TSH-Rezeptor-Präparationen Präparationen von geeigneten rekombinanten humanen TSH-Rezeptoren sowie selektive monoklonalen Antikörper zu ihrer Immobilisierung verwendet werden, werden Autoantikörper bei im wesentlichen alle Patienten korrekt nachgewiesen (S. Costagliola et al., J. Clin. Endocrinol Metab 84:90-97, 1999).

Es ist allgemein bekannt, daß bei der als Morbus Basedow (englisch: Graves' disease) bekannten Schilddrüsen-Autoimmunerkrankung stimulierende Autoantikörper (TSAb) eine Rolle spielen, die gegen der TSH-Rezeptor gebildet werden und mit diesem so wechselwirken, daß die Schilddrüse stimuliert wird, was sich als Schilddrüsenüberfunktion (Hyperthyreose) äußert. Ein charakteristisches klinisches Begleitsymptom des Morbus Basedow, das nicht unmittelbar auf die Hyperthyreose zurückzuführen ist, ist der sogenannte Exophthalmus ("Glotzauge"; endokrine Orbitopathie oder Ophthalmopathie oder EO; englisch: Graves' ophthalmopathy oder GO). Die klinischen Folgen dieser Begleiterkrankung reichen von eher kosmetischen Störungen (Glotzaugen) bis zum Verlust der Sehfähigkeit. Eine als gut begründbar angesehene und daher gegenwärtig intensiv untersuchte Annahme geht davon aus, daß die für die Stimulation der Schilddrüse verantwortlichen krankheitstypischen Autoantikörper auch an TSH-Rezeptoren oder Varianten oder Fragmente davon binden, die im retrookularen Gewebe nachgewiesen werden konnten, was über komplexe Zusammenhänge zu einer immunogenen Entzündung des Augen-Muskelgewebes und zu dem typischen Krankheitsbild einer EO führen könnte (vgl. z.B. A.P.Weetman, Molecular and Cellular Endocrinology 126 (1997) 113-116).

Es sind ferner Fälle von Hyperthyreose bekannt, die mit aktivierenden Mutationen des TSH-Rezeptors in Verbindung gebracht werden (vgl. Thyroid, Vol. 8, 1998, 559-564 und die darin zitierte einschlägige Literatur), insbesondere beim sogenannten autonomen Schilddrüsen-Adenom.

Zur Therapie des Morbus Basedow stehen derzeit drei auf unterschiedlichen Prinzipien beruhende Behandlungsmöglichkeiten zur Verfügung:
a) medikamentöse Behandlung mit Thioharnstoffderivaten (z.B. Carbimazol (INN), Methimazol (Thiamazol; INN));
b) teilweise Zerstörung von Schilddrüsengewebe durch Gabe von radioaktivem Jod, und
c) chirurgische Entfernung des Schilddrüsengewebes.

In Deutschland, vielen Ländern Europas sowie Japan ist die medikamentöse Therapie gemäß a) beim erstmaligen Auftreten des Morbus Basedow die Regeltherapie. In speziellen Situationen (z.B. große Struma, zusätzlicher Verdacht auf Autonomie) kann auch sofort auf eine der sogenannten definitiven Therapien nach b) oder c) zurückgegriffen werden. In den USA ist eine solche Therapie nach b) und c) aus historischen Gründen eher die Regel, und es werden nur sehr wenige Patienten (< 20%) medikamentös gemäß a) therapiert.

Die medikamentöse Therapie hat Vor- und Nachteile. Der Vorteil ist, daß der Patient durch die befristete Gabe von Medikamenten in einen Zustand gebracht werden kann, in dem die Schilddrüsenfunktion normalisiert ist (Euthyreose), und der Morbus Basedow tritt in eine sogenannte Remission ein. Im günstigsten Falle benötigt der Patent nach einiger Zeit keine weitere Medikation, hat jedoch noch immer eine voll funktionsfähige Schilddrüse, was eine Substitutionstherapie mit Schilddrüsenhormonen, wie sie nach den definitiven Therapien gemäß b) und c) erforderlich ist, überflüssig macht.

Diese sogenannten definitiven Therapien führen in den meisten Fällen dazu, daß keine voll funktionsfähige Schilddrüse mehr vorhanden ist, und zum Ausgleich der dadurch hervorgerufenen Hypothyreose ist eine oft lebenslange Substitutionstherapie mit Schilddrüsenhormonen notwendig.

Ein wesentliches Problem der Entscheidung für die richtige Therapie ist der zu erwartende Erfolg. Prinzipiell können bei allen drei genannten derzeit bekannten Therapieformen Erfolge erzielt werden, d.h. die Remission der Erkrankung kann für sehr lange, vielleicht sogar für immer, erreicht sein. Bei einem Erfolg der Therapie gemäß a) wären dann keinerlei zusätzliche therapeutische Maßnahmen mehr notwendig. Im Falle von b) und c) müssen jedoch meist die fehlenden Schilddrüsenhormone medikamentös von außen zugeführt werden.

Ein gemeinsames Problem aller Therapieformen ist jedoch, daß es mehr oder weniger häufig zu sogenannten Rezidiven kommt. Das heißt, nach einer gewissen Zeit nach der vermeintlich erfolgreichen Therapie bricht die Erkrankung erneut aus, was neuerliche therapeutische Eingriffe notwendig macht. Obwohl derartige Rezidive bei allen drei Therapieformen vorkommen können, ist die medikamentöse Therapie gemäß a) davon am häufigsten betroffen. So können nur ca. 50% aller Morbus Basedow-Patienten durch eine ein- bis zweijährige Gabe von Medikamenten (Tabletten) für eine lange Folgeperiode in Remission gebracht werden.

Die wegen Erhaltung der Schilddrüse an sich zu bevorzugende Therapie mit Medikamenten hat zusätzliche Nachteile. Zum einen tritt die Wirkung der Medikamente nur mit einer zeitlichen Verzögerung auf, da sie im wesentlichen die Neusynthese von Hormonen hemmen, nicht jedoch die Freisetzung von bereits in der Schilddrüse gebildeten und gespeicherten Hormonen. Es dauert somit ein bis zwei Wochen, bis eine Wirkung der Medikamente zu erkennen ist. Bei hochgradig kranken Patienten (thyreotoxische Krise) kann das dazu führen, daß die Behandlung versagt. Ähnlich verhält es sich mit der Gabe von Radiojod. Auch hier setzt der therapeutische Effekt nur verzögert ein.

Zusätzlich kommt es in wenigen seltenen Fällen bei der medikamentösen Behandlung zu Nebenwirkungen, die ein sofortiges Absetzen der Medikamente erfordern (Agranulozytose) Bleibt der Patient dann noch hyperthyreot, was in der Regel der Fall ist, gibt es nur noch wenige therapeutische Möglichkeiten. Durch die Nebenwirkung der Agranulozytose (kompletter Verlust der weißen Blutzellen) ist er außerordentlich infektionsgefährdet und nur bedingt operationsfähig.
Ein Rückgriff auf die alternativen Therapieansätze b) und c) scheidet daher in der Regel aus. Es besteht somit grundsätzlich ein Bedarf nach weiteren therapeutischen Behandlungsmöglichkeiten des Morbus Basedow, insbesondere nach solchen, die eine Chance bieten, die Schilddrüse zu erhalten und das Krankheitsgeschehen ursächlich zu beeinflussen.

Durch die Verwendung von blockierenden Antikörpern gegen den TSH-Rezeptor gemäß den Patentansprüchen 1 bis 4 wird eine derartige weitere Therapiemöglichkeit geschaffen, die sich neben den bisherigen therapeutischen Behandlungen als vierte Therapieform etablieren könnte und erhebliche Vorteile bieten kann, auf die weiter unten noch näher eingegangen wird.

Eine derartige Therapie wird dadurch ermöglicht, daß selektiv blockierende Antikörper gegen den TSH-Rezeptor in Form monoklonaler Antikörper hergestellt werden konnten und gezeigt werden konnte, daß diese Antikörper tatsächlich den stimulierenden Effekt von Schilddrüsen-stimulierenden Autoantikörpern (TSAb) aufheben können.

Die vorliegende Erfindung betrifft daher gemäß den Ansprüchen 5 bis 7 auch derartige monoklonale blockierende Antikörper, für die eine Herstellungsmöglichkeit sowie eine Selektionsmöglichkeit nachfolgend im experimentellen Teil beschrieben wird und die vorsorglich zusätzlich durch Hinterlegung unter den Bezeichnungen 4E9/B2/C1 (DSM ACC2389); 4C1/E1/G8 (DSM ACC2390); 1B1/E10/B11/C12 (DSM ACC2391); 7E3/F8/E3 (DSM ACC2392); 3H10/A11/A1 (DSM ACC2393) am 19.2.1999 bei DSMZ, Braunschweig durch Hinterlegung nach den Bestimmungen des Budapester Vertrags der Öffentlichkeit zugänglich gemacht wurden.

An den TSH-Rezeptor bindende blockierende Antikörper können in Form monoklonaler Antikörper nach der im experimentellen Teil beschriebenen Arbeitsweise erzeugt und selektiert werden, insbesondere nachdem die relativ kurze Aminosäuresequenz der extrazellulären Domäne des TSH-Rezeptors identifiziert wurde, an die derartige blockierende Antikörper bzw. bestimmte, sehr wirksame derartige Antikörper binden.

Wie ebenfalls im experimentellen Teil näher beschrieben wird, konnte gezeigt werden, daß im eingangs charakterisierten Bioassay unter Verwendung von JP09CHO-Zellen die gleichzeitige Zugabe von derartigen monoklonalen Antikörpern die Stimulation dieser Zellen durch TSH, erkennbar an der cAMP-Bildung, selektiv unterdrücken kann.

Das gleiche gilt für den Fall, daß die Stimulation der genannten Zellen im Bioassay nicht mit TSH erfolgt, sondern durch Zugabe von Seren von Morbus Basedow-Patienten mit einem hohen Titer an stimulierenden Autoantikörpern (TSAb).

Die Verfügbarkeit derartiger selektiver blockierender Antikörper gegen den TSH-Rezeptor ermöglicht es, eine neuartige Therapie des Morbus Basedow ins Auge zu fassen, die durch die Verwendung derartiger blockierender Antikörper bzw. die Verwendung derartiger Antikörper zur Herstellung von Arzneimitteln und die Verabreichung derartiger Arzneimittel, üblicherweise per Injektion oder Infusion, gekennzeichnet ist.

Als Vorteile einer derartigen Therapie mit blockierenden Antikörpern können genannt werden:
a) Es ist zu erwarten, daß durch die Verabreichung der blockierenden Antikörper, die schnell und mit hoher Effizienz an den TSH-Rezeptor binden und diesen blokkieren, eine rasche Hemmung der Stimulation des TSH-Rezeptors erreichbar ist.
b) Die Therapie des Morbus Basedow erfolgt direkt am Wirkungsort der Erkrankung und ist somit als eine ursächliche Therapie anzusehen.
c) Die Therapie mit den Antikörpern kann auch bei schwerkranken Patienten angewandt werden, bei denen die bisher bekannten Therapieformen versagen, ohne daß die in solchen Fällen gegen die bekannten Therapieformen bestehenden Bedenken zum Tragen kommen.
d) Da durch die blockierenden Antikörper das Zielorgan des autoimmunen Angriffs ruhiggestellt werden kann, erscheint in Analogie zu Beobachtungen von Beeinflussungsmöglichkeiten autoimmuner Prozesse bei anderen Organen eine Abschwächung des autoimmunen Prozesses im Bereich der Möglichkeiten.
e) Ähnlich wie bei der medikamentösen Behandlung kann die Therapie zeitlich begrenzt mit der Option auf vollständige Remission und Erhaltung des Organs durchgeführt werden.

Vom Wirkungsprinzip her sind keine gravierenden Nebenwirkungen zu erwarten.

Die obigen Vorteile sollen noch etwas ausführlicher erläutert werden.

Ein therapeutischer Ansatz unter Verwendung von Antikörpern, die den TSH-Rezeptor blockieren, verspricht einen unmittelbaren Therapieerfolg ohne die zeitlichen Verzögerungen, die bei der Verabreichung von Medikamenten bzw. Radiojod beobachtet werden. Die Gabe von blockierenden Antikörpern erscheint auch möglich, wenn eine sofortige Operation eines hypothyreoten Patienten nicht in Frage kommt. Sie kann daher zur einzigen Option für eine schnelle Normalisierung der Stoffwechsellage werden.

In den seltenen Fällen, bei denen es bei der medikamentösen Therapie zu einer Agranulozytose kommt und derzeit keine weiteren therapeutischen Eingriffsmöglichkeiten mehr existieren, kann der Einsatz von blockierenden Antikörpern eine neue, unter Umständen lebensrettende Behandlungsalternative darstellen.

Da bei Morbus Basedow die Einwirkung von stimulierenden Autoantikörpern auf den TSH-Rezeptor für das Entstehen der meisten klinischen Symptome ursächlich verantwortlich ist, wird durch die therapeutische Verabreichung von blockierenden Antikörpern, die mit diesen krankmachenden stimulierenden Autoantikörpern konkurrieren, die einmalige Chance eröffnet, das Krankheitsgeschehen direkt am Krankheitsort ursächlich zu bekämpfen. Eine derartige Vorgehensweise mit einer therapeutischen Intervention dort, wo eine Krankheit ihren Ursprung hat, ist nur sehr selten möglich.

Ein weiterer Vorteil besteht in der zu erwartenden Möglichkeit, daß die Therapie mit blockierenden Antikörpern nur zeitlich begrenzt durchgeführt werden muß, und daß nach Abschluß der Therapie die Schilddrüse erhalten bleibt. Es ist bekannt, daß der Autoimmunprozeß in vielen Fällen von Morbus Basedow-Erkrankungen zeitlich begrenzt ist, d.h. daß die Krankheit in vielen Fällen nach einiger Zeit abklingt. Die Verabreichung von blockierenden Antikörpern ermöglicht die Überbrückung der akuten Krankheitsphase mit klinisch relevanten Symptomen, ist also ähnlich wie die derzeitige medikamentöse Therapie von vorübergehendem Charakter.

Es besteht darüber hinaus Grund zu der Hoffnung, daß die Zufuhr von blockierenden Antikörpern immunmodulatorisch wirkt und zu einer Abschwächung oder sogar Heilung des Autoimmunprozesses führen kann. Durch die Blockierung der Wirkung der krankmachenden stimulierenden Antikörper sowie auch die Blockierung des physiologischen TSH kommt es zu einer vollständigen Stilllegung der Schilddrüse. Eine derartige Stilllegung der Schilddrüse, bei der deren Stoffwechsel stark vermindert wird, führt zu einer verminderten Expression von Antigenen oder MHCII-Molekülen, was wiederum zu einer verminderten Stimulation des Immunsystems und einer Abschwächung der Immunantwort führen kann.

Daß eine derartige Hoffnung vernünftig ist, ergibt sich aus Beobachtungen, die bei anderen Autoimmunerkrankungen gemacht werden konnten. So konnte im Falle der Autoimmunerkrankung des Diabetes mellitus Typ I gezeigt werden, daß die frühe Gabe von Insulin, die dazu führt, daß die körpereigene Insulinproduktion herabgesetzt werden kann, bei einer Gruppe von Patienten mit relativ langsamem Krankheitsverlauf vorteilhaft ist (vgl. Kobayashi et al., Diabetes 45, 622 (1996)). Die frühe Insulingabe führt zu einer Schonung der Insulin produzierenden Zellen der Langerhansschen Inseln, die weniger aktiv sind und weniger rasch durch den Autoimmunangriff zerstört werden und somit länger körpereigenes Insulin produzieren können. Es kann angenommen werden, daß eine Stilllegung der Schilddrüse mittels blockierender Antikörper ähnliche positive Wirkungen haben kann.

Ferner besteht die begründete Hoffnung, daß die Gabe der blockierenden Antikörper auch die immunogene Entzündung direkt positiv beeinflussen kann, die für das Begleitsymptom des Morbus Basedow, die endokrine Orbitopathie (EO), verantwortlich ist. Bisher kann die EO nicht kausal therapiert werden. Neben rein symptomatischen Behandlungen mit z.B. getönten Augengläsern, Augentropfen kommen in schwereren Fällen agressivere Behandlungen zur Anwendung, und zwar in Form einer medikamentösen Therapie mit Glucocorticoiden, einer Hochvoltbestrahlung der Orbita (Augenhöhle) oder einer operativen Dekompression der Orbita. Die Behandlungserfolge der o.g. Therapien sind jedoch unbefriedigend. Bevor eine solche Therapie überhaupt zur Anwendung kommen kann, sollte der Patient jedoch in jedem Falle euthyreot sein. Die Herstellung einer euthyreoten Stoffwechsellage durch blockierende Antikörper gemäß der vorliegenden Erfindung unterstützt daher auch die Therapie einer EO, und die frühe Verabreichung von solchen Antikörpern an Morbus Basedow Patienten könnte auch eine präventive Wirkung auf die Entwicklung der Augensymptomatik einer EO haben.

Die blockierenden Antikörper können ferner auch zur Behandlung von bestimmten Hyperthyreosen eingesetzt werden, die nicht, wie beim Morbus Basedow, auf eine Stimulation der Schilddrüse durch Autoantikörper zurückzuführen sind, sondern auf eine Überstimulation der Schilddrüse durch TSH. Zu nennen ist hierbei z.B. eine Hypophysenüberfunktion mit erhöhter TSH-Ausschüttung aufgrund eines Hypophysenadenoms.

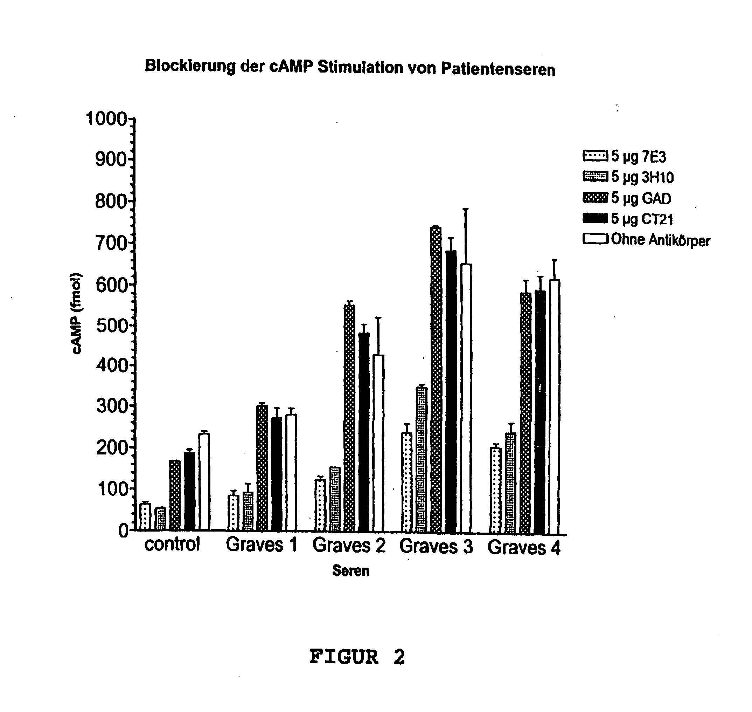

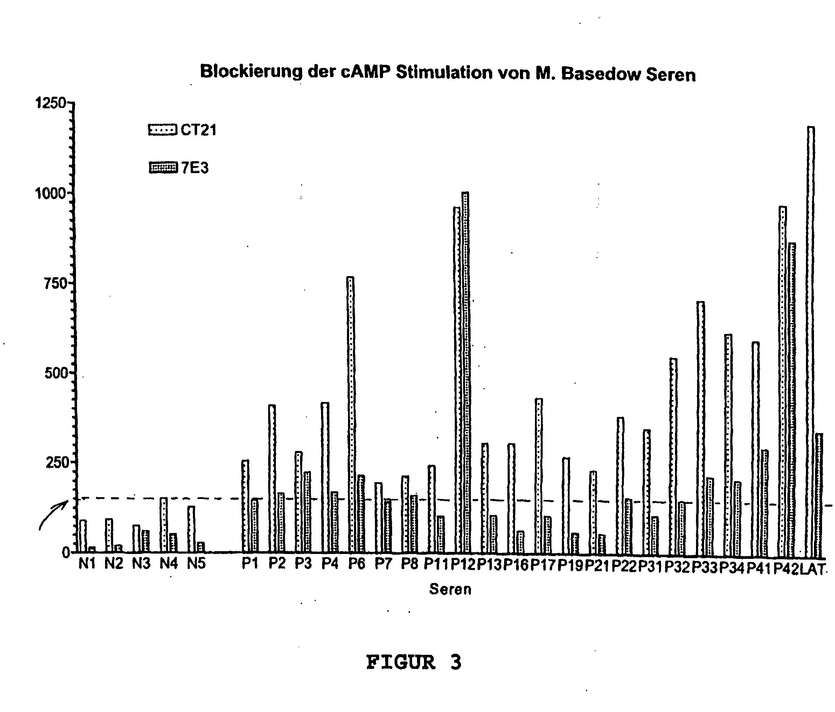

Ferner erscheint auch die Behandlung einer Hyperthyreose, die mit aktivierenden Mutationen des TSH in Verbindung zu bringenist, mit den erfindungsgemäß zuverwendenden blockierenden Antikörpern vielversprechend. Hierbei ist v.a. die Beobachtung von Bedeutung, daß die blockierenden Antikörper nicht nur die Schiddrüsenfunktion in Gegenwart stimulierender Autoantikörper vermindern, sondern auch bei Normalpersonen eine Herabsetzung der Schilddrüsenfunktion beobachtet wird (vgl. Fig.2 der vorliegenden Anmeldung, insbesondere die Werte für die Kontrollen in Gegenwart der Antikörper 7E3 und 3H10 im Vergleich zu der Kontrolle ohne Antikörper bzw. die Werte für die Normalseren in Fig.3).

Besondere Bedenken gegen eine Therapie mit blockierenden Antikörpern bestehen nicht. Zu Therapien mit Antikörpern, die prinzipiell als natürliche Substanzen anzusehen sind, existieren es umfangreiche klinische Erfahrungen. Es ist ferner bekannt, daß blockierende Antikörper, wie sie im Rahmen der neuartigen Therapie verwendet werden sollen, bei vielen Morbus Basedow-Patienten vorkommen, ohne daß das Vorkommen derartiger blockierender Antikörper mit irgendwelchen gesundheitsschädlichen Auswirkungen korreliert werden kann.

Die blockierenden Antikörper, deren Herstellung und Eigenschaffen im experimentellen Teil gezeigt werden, sind aufgrund der zur Anwendung kommenden Hybridomtechnik keine menschlichen Antikörper sondern tierische (Maus) Antikörper. Diese können normalerweise nicht unmittelbar zur Therapie in der Humanmedizin verwendet werden. Es gehört jedoch heutzutage zu den etablierten Techniken, derartige tierische Antikörper für eine Verwendung in der Humanmedizin zu "humanisieren" (vgl. z.B. B.R.Glick, J.J.Pasternak, Molekulare Biotechnologie, Spektrum Akademischer Verlag Heidelberg, Berlin, Oxford, 1995, S. 243 ff). Dabei werden die variablen Fv-Fragmente der tierischen Antikörper mit den konstanten Domänen und Fc-Fragmenten von humanen Antikörpern gekoppelt, was dazu führt, daß derartige humanisierte (vermenschlichte oder chimäre) Antikörper vom menschlichen Immunsystem nicht mehr als artfremde Strukturen erkannt und angegriffen werden.

Die blockierenden Antikörper, deren Gewinnung und Herstellung im experimentellen Teil gezeigt wird und mit denen nachgewiesen wurde, daß diese die Wirkungen von TSH und stimulierenden Antikörpern (TSAb) aufheben können, sind blockierende monoklonale Antikörper mit bestimmten Eigenschaften. Sie erscheinen derzeit, in humanisierter Form, aufgrund Ihrer Spezifität und hohen Affinität für den TSH-Rezeptor für die erfindungsgemäße Verwendung zur Therapie des Morbus Basedow besonders geeignet. Das soll jedoch die Verwendung anderer, im gleichen Sinne blockierend wirkender Antikörpern gegen den TSH-Rezeptor nicht als nicht unter die vorliegende Erfindung fallend ausschließen. So ist nicht ausgeschlossen, daß blockierende Antikörper mit vergleichbaren Eigenschaften existieren, die an andere Aminosäuresequenzen des TSH-Rezeptors, insbesondere seiner extrazellulären Domäne, binden und daher ebenfalls eingesetzt werden können. Ferner können auch polyklonale Antikörper, die mit gleicher Wirkung an eine oder mehrere Sequenzen des TSH-Rezeptors binden, zur Anwendung kommen. Die Antikörper können ferner tierische bzw. humanisierte tierische Antikörper sein, können jedoch auch humane Antikörper sein, die z.B. durch selektive Isolierung aus geeigneten Patientenseren gewonnen wurden, oder humane monoklonale Antikörper, wie sie z.B. durch die Technik der sog. EBV-Transformierung hergestellt weren können (vgl. N.G.Morgenthaler et al., J.Clin.Endocrinol.Metab. 81: 3155-3161, 1996) Derartige Antikörper können auch in Form ihrer spezifisch an den TSH-Rezeptor bindenen Fragmente, z.B. Fab oder Fab' Fragmente ohne Fc-Teil, eingesetzt werden. Ferner können die Antikörper oder Antikörperfragmente auch in Form geeigneter Konjugate, gebunden an Carrier-Moleküle, die inert sein können, die jedoch auch zusätzliche therapeutische Aufgaben erfüllen können, eingesetzt werden. Auch Gemische von verschiedenen derartigen Antikörpern, z.B. Kombinationen von verschiedenen monoklonalen Antikörpern, sind prinzipiell verwendbar.

Die erfindungsgemäß zu verwendenden blockierenden Antikörper sind solche, die durch Bindung an den extrazellulären Teil des TSH-Rezeptors die Wirkung von TSH und stimulierenden TSH-Rezeptor-Autoantikörpern aufheben. Sie weisen eine ausreichend hohe Affinität zum TSH-Rezeptor auf, um in für einen therapeutischen Einsatz geeigneten Konzentrationen erfolgreich TSH und stimulierende Autoantikörper von diesem TSH-Rezeptor verdrängen bzw. fernhalten zu können.

Die erfindungsgemäß zu verwendenden Autoantikörper werden in einer Form verabreicht, wie sie für Antikörpertherapien typisch ist. Üblicherweise werden Injektionslösungen, gegebenenfalls Infusionslösungen, zur Verfügung gestellt, die die für die Behandlung vorgesehenen blockierenden Antikörper in einem wäßrigen, physiologischen Trägermedium enthalten. Das Medium kann die für derartige Medien üblichen schützenden oder stabilisierenden Zusätze sowie gegebenenfalls auch nährende oder den Behandlungserfolg auf andere Weise fördernde Zusätze enthalten. Die Anwesenheit weiterer Wirkstoffe, insbesondere solcher mit einem Einfluß auf das Immungeschehen, wird ausdrücklich als mögliche Ausführungsvariante der erfindungsgemäßen Verwendung blockierender Antikörper zur Behandlung des Morbus Basedow angesehen.

Die für eine individuelle Behandlung empfehlenswerte oder erforderliche Menge und Konzentration der zu verabreichenden blockierenden Antikörper ist gegebenenfalls unter Berücksichtigung des individuellen Krankheitszustands des Patienten festzulegen. So können schwere Krankheitserscheinungen bzw. hohe Titer an TRAb einen Einsatz höherer Mengen und Konzentrationen der erfindungsgemäß zu verwendenden Antikörper erfordern als schwächer ausgeprägte Krankheitssymptome mit niedrigeren Autoantikörpertitern. Geeignete Mengen und Konzentrationen können anhand der Beobachtung des Therapieerfolges empirisch festgelegt werden, ohne daß diesbezüglich besondere Probleme zu erwarten wären.

Nachfolgend wird die Erfindung ergänzend durch Versuchsergebnisse erläutert, die die Gewinnung, Isolierung und, unter Bezugnahme auf drei Figuren, die Prüfung von monoklonalen blockierenden Antikörpern gegen den TSH-Rezeptor erläutern, wie sie, gegebenenfalls nach einer an sich bekannten Humanisierung, zur Grundlage der erfindungsgemäßen Verwendungen gemacht werden können.

In den Figuren zeigen:
- Figur 1: die spezifische Unterdrückung der cAMP-Bildung von JP09 CHO Zellen durch fünf monoklonale blockierende Antikörper in Gegenwart von bovinem TSH im Bioassay;
- Figur 2: die spezifische Unterdrückung der cAMP-Bildung von JP09 CHO Zellen durch zwei monoklonale blockierende Antikörper in Gegenwart von 4 Morbus Basedow Seren im Bioassay; und
- Figur 3: die spezifische Unterdrückung der cAMP-Bildung von JP09 CHO Zellen durch einen monoklonalen blockierenden Antikörper in Gegenwart von 21 Morbus Basedow Seren sowie des LATS Standards im Bioassay.

### Erzeugung und Charakterisierung von monoklonalen blockierenden Antikörpern gegen den humanen TSH-Rezeptor

Die Erzeugung, Selektion und Charakterisierung von erfindungsgemäß verwendbaren monoklonalen Antikörpern gegen den humanen TSH-Rezeptor erfolgte weitgehend unter Verwendung vorbeschriebener Materialien und Techniken. Eine detaillierte Beschreibung derartiger Materialien und Techniken wurde im nachfolgenden experimentellen Teil durch einen Verweis auf die einschlägigen Literaturstellen ersetzt, soweit dadurch das Verständnis und die Nacharbeitbarkeit nicht ernsthaft beeinträchtigt wird.

### Immunisierung und Erzeugung von Hybridomas

Die extrazelluläre Domäne (ECD) des humanen TSH-Rezeptors (Aminosäuren 1-415) wurde in Form eines Fusionsproteins mit Glutathion-S-transferase in einem prokaryontischen Expressionssystem hergestellt (vgl. Harfst, E. et al., J. Mol.Endocrinol. 9, 1992, S.227-236).

Mit dem erhaltenen prokaryontisch exprimierten ECD-Glutathion-S-transferase-Fusionsprotein wurden BALB/c-Mäuse immunisiert, und die Herstellung und das Klonieren von Hybridomas erfolgte so wie in näheren Einzelheiten beschrieben ist in Johnstone, A.P. et al., Mol.Cell.Endocrinol. 105, 1994, R1-R9.

Die Überstände der klonierten Hybridomas wurden mittels "Flow cytofluorimetry" gemäß der Arbeitsweise gescreent, die ebenfalls beschrieben ist in Johnstone, A.P. et al., Mol.Cell.Endocrinol. 105, 1994, R1-R9, wobei als Bindungsreagens die vorbeschriebenen Zelllinien "FLD4" und "FLEA.2" verwendet wurden. Diese Zelllinien sind abgeleitet von transformierten CHO-K1-Zellen und exprimieren unterschiedliche Mengen des funktionalen humanen TSH-Rezeptors voller Länge, gekoppelt an Adenylatcyclase, wobei das vervielfältigbare Glutaminsynthetase-Expressionssystem zur Anwendung kommt, wie detailliert beschrieben ist in Harfst, E. et al., Mol. Cell. Endocrinol. 83, 1992, S.117-123, und in Harfst, E., Johnstone, A.P.,, Anal. Biochem. 207, 1992, S.80-84.

### Reinigung von monoklonalen Antikörpern

Hybridomas wurden in Spinner-Kulturen in UltraDoma-PF (Bio-Whittaker, Walkerville, Maryland, USA), enthaltend 2% FCS und 100 IU/ml Streptomycin, gezüchtet. Antikörper wurden aus den Überständen der Gewebekulturen mittels Affinitätschromatographie an Protein A (mit den für IgG1 geeigneten hohen Salz- und pH-Puffer-Modifikationen) isoliert, wie beschrieben ist in Johnstone, A.P., Thorpe, R., 1996, Immunochemistry in Practice, 3. Auflage, Blackwell Science Ltd., Oxford.

### Charakterisierung der Antikörper verschiedener Hybridomas

Die Antikörper wurden durch Immunpräzipitation mit Zellen von den hTSH-Rezeptor exprimierenden rekombinanten Zelllinien nach der in vivo Markierung des Rezeptorproteins mit [³⁵S]-Methionin, sowie ferner durch Immun- (bzw. Protein-) Blotting mit der extrazellulären Domäne des hTSHR (vgl. Harfst E., et al., J.Mol.Endocrinol., 9, 1992, 227-236) auf ihr Bindungsverhalten gegenüber dem rekombinanten hTSH-Rezeptor untersucht. Beim Immun-Blotting wurden die blokkierten Membranen mit extrazellulären Domänen des hTSH-Rezeptors für 1-2h in Hybridoma-Kulturüberstand, auf ein Viertel verdünnt in 200 mM NaCl, 50 mM Tris-HCl, pH 7,4, inkubiert und dann gewaschen. Gebundene Antikörper wurden mittels eines zweiten Antikörpers (Peroxidase-Konjugat eines Anti-Maus Immunglobulins) und eines handelsüblichen Chemilumineszenz-Systems nachgewiesen und aufgrund ihrer Reaktion mit dem rekombinanten hTSH-Rezeptor selektiert.

Die selektierten monoklonalen Antikörper wurden mittels eines cAMP-Bioassays (Page, S.R. et al., J. Endocrinol. 126, 1990, S. 333-340) sowie in einem Radioliganden-Assay im Hinblick auf ihre Fähigkeit., mit radioaktiv markiertem bovinem TSH um die von den Zelllinien "FLD4" und "FLEA.2" (s.o.) exprimierten hTSH-Rezeptoren zu kompetieren (vgl. Harfst, E. et al., Mol. Cell. Endocrinol. 83, 1992, S.117-123, und Harfst, E., Johnstone, A.P.,, Anal. Biochem. 207, 1992, S.80-84), näher charkterisiert. Dazu wurden Hybridoma-Kulturüberstände bzw. die daraus durch Reinigung gewonnenen IgG-Fraktionen (gereinigten monoklonalen Antikörper) und radioaktiv markiertes bovines TSH mit den genannten Zellen inkubiert, wonach die daran gebundene Radioaktivität ermittelt wurde. Die untersuchten Antikörper inhibierten die TSH-Bindung.

### Ermittlung der zugehörigen Antikörper-bindenden Epitope des hTSH-Rezeptors

Die Bindungsstellen (Epitope) der selektierten monoklonalen Antikörper an die extrazelluläre Domäne des hTSHR wurden unter Verwendung einer Vielzahl von überlappenden kurzen synthetischen Teilpeptiden aus der ECD des hTSH-Rezeptors auf Cellulose-Membranen mit Hilfe eines kommerziellen Kits (SPOTS Kit, Genosys) genauer bestimmt.

Dabei zeigte sich, daß alle aufgrund ihrer Bindung an den hTSH-Rezeptor selektierten Antikörper mit Aminosäuresequenzen aus dem Bereich der Aminosäuren 335-390 des humanen TSH-Rezeptors reagierten, und daß außerdem keiner der Antikörper im cAMP-Bioassay die cAMP-Bildung von rekombinanten hTSHR-Zelllinien stimulierte. Fünf selektierte Antikörper von stabilen Hybridomas reagierten mit einer kurzen Peptidsequenz mit den vier Aminosäuren 381-384 des humanen TSH-Rezeptors (FDSH bzw. Phe Asp Ser His), wobei die erhaltene Bindung verstärkt werden kann, wenn die kurzen Peptide zusätzlich bis zu drei der angrenzenden Aminosäuren 385 bis 387 (YDY bzw. Tyr Asp Tyr) bzw. 378 bis 381 (LQA bzw. Le Gln Ala) aufwiesen. Diejenigen der selektierten Antikörper mit den höchsten Affinitäten für den hTSH-Rezeptor inhibierten im o.g. kompetitiven Radioligandenassay bei 1 µg/ml die Bindung des radioaktiven bovinen TSH zu 80-90% und bei 0,1 µg/ml immer noch zu mehr als 50%.

In Form von geeignet markierten oder fixierten Peptiden, die der o.g. Sequenz 378-387 bzw. einer Teilsequenz davon entsprechen, stehen nunmehr auch Mittel zur Verfügung, die es erheblich erleichtern können, in Hybridoma-Überständen von bei der Nacharbeitung der o.g. Arbeitsweisen erhaltenen Klonen solche monoklonalen Antikörper zu identifizieren, die ein geeignetes Bindungsverhalten im Sinne der vorliegenden Erfindung aufweisen.

### Inhibierung der Bindung von TSAb aus Seren von Morbus Basedow Patienten

Fünf monoklonale Antikörper, die bei den vorstehend beschriebenen Versuchen erzeugt wurden, die an die o.g. Aminosäuresequenz 381-384 des hTSH-Rezeptors binden und die nachfolgend mit 1B1, 7E3, 4E9, 3H10 und 4C1 bezeichnet werden, wurden auf ihre Eignung untersucht, im eingangs erwähnten Bioassay (N.G. Morgenthaler et al. in Horm. Metab. Res. 30 (1998), S. 162-168) die Bindung von bovinem TSH und von stimulierenden Autoantikörpern aus den Seren von Morbus Basedow Patienten an einen rekombinant exprimierten hTSH-Rezeptor, erkennbar an einer Stimulation der cAMP-Produktion, zu unterdrücken.

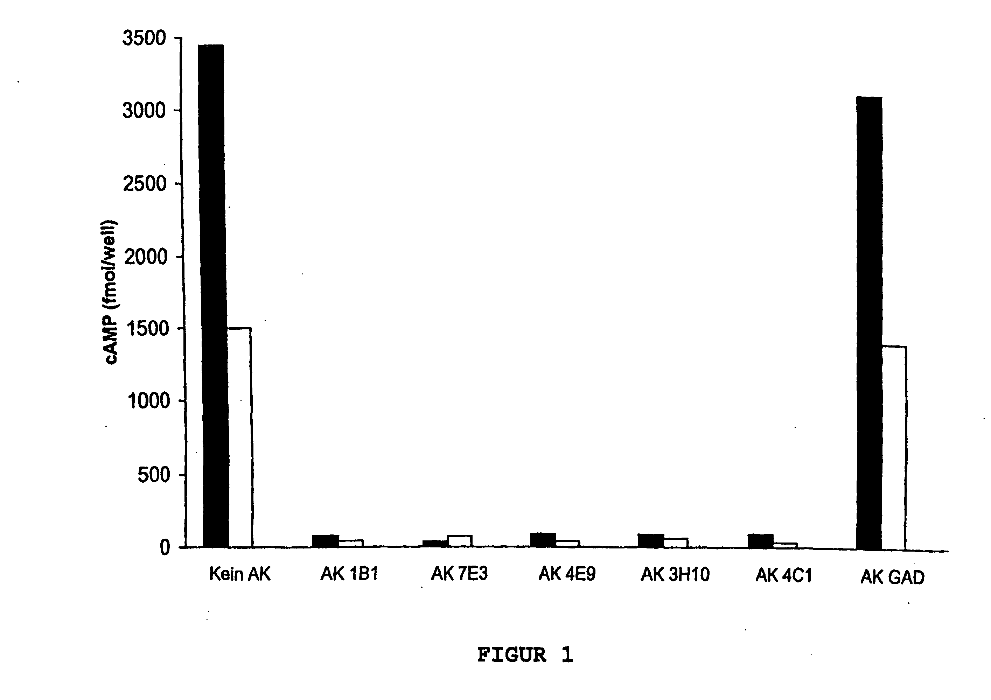

Dazu wurden JP09 CHO-Zellen jeweils mit 1 µU (Fig.1, schwarze Balken) bzw. 10 µU (Fig.1, weiße Balken) bovinem TSH pro Well und Zugabe des jeweiligen zu prüfenden Antikörpers inkubiert. Zur Kontrolle wurde auch ein andersartiger Antikörper (gegen Glutamatdecarboxylase; GAD) eingesetzt, der keinerlei Wirkung zeigte. Die Ergebnisse sind in Fig.1 gezeigt, und es ist klar zu erkennen, daß alle fünf untersuchten Antikörper die Stimulation der cAMP-Produktion unterdrücken.

Der Versuch wurde mit den Antikörpern 7E3 und 3H10 sowie zwei andersartigen Antikörpern (GAD; CT21) wiederholt, wobei jedoch anstelle des bovinen TSH Seren von 4 verschiedenen Morbus Basedow Patienten zur Stimulation der cAMP-Produktion eingesetzt wurden. Die Ergebnisse sind in Fig.2 gezeigt. Es ist deutlich zu erkennen, daß in Gegenwart der Antikörper 7E3 und 3H10 die Bildung von cAMP deutlich unterdrückt ist.

In einem weiteren Versuch wurde im gleichen Bioassay die Unterdrückung der cAMP Produktion durch den Antikörper 7E3 in Gegenwart von 5 Normalseren (N), 21 Seren von Morbus Basedow Patienten (P) und von LATS (internationaler Standard - long acting thyroid stimulator) bestimmt. Zum Vergleich wurden wiederum die Ergebnisse in Gegenwart eines andersartigen Antikörpers (CT21; monoklonaler Antikörper gegen Procalcitonin) gemessen. Die Ergebnisse sind in Fig.3 gezeigt. Es ist wiederum deutlich zu erkennen, daß der Antikörper 7E3 in 20 von 22 Fällen die Bildung von cAMP wirksam unterdrückt.

Im Hinblick auf die Möglichkeit, mit blockierenden Antikörpern Hyperthyreosen aufgrund aktivierender Mutationen zu behandeln, wird auf die Werte für die Kontrollseren bzw. Normalseren in den Figuren 2 und 3 verwiesen: In allen Fällen war die cAMP-Produktion der Testzellen in Gegenwart der blockierenden Antikörper im Vergleich mit antikörperfreien Seren bzw. gegenüber Seren mit "andersartigen" Antikörpern erkennbar herabgesetzt. Das deutet auf eine Wirkung der blockierenden Antikörper hin, die über die reine Verhinderung der Stimulierung der Schilddrüse durch TSH bzw. stimulierende Autoantikörper hinausgeht.

## Patentansprüche

1. Verwendung von blockierenden Antikörpern gegen den humanen TSH-Rezeptor (hTSH-Rezeptor), die eine kurze Peptidsequenz erkennen, die die Aminosäuren FDSH (Phe Asp Ser His) der Positionen 381-384 des hTSH-Rezeptors sowie gegebenenfalls zusätzliche flankierende Aminosäuren aus dessen extrazellulärer Domäne aufweist, oder ihrer spezifisch bindenden Fragmente zur Herstellung von Arzneimitteln zur Behandlung von Hyperthyreosen, die auf stimulierende Autoantikörpern gegen den hTSH-Rezeptor (Morbus Basedow) oder eine Überstimulation der Schilddrüse durch endogenes TSH oder auf aktivierende Mutationen des hTSH-Rezeptors zurückzuführen sind, und von Arzneimitteln zur Behandlung der endokrinen Orbitopathie.

2. Verwendung nach Anspruch 1 zur Herstellung von per Injektion oder Infusion zu verabreichenden Arzneimitteln.

3. Verwendung nach einem der Ansprüche 1 oder 2, bei der der zu verwendende blockierende Antikörper ein monoklonaler humaner oder tierischer Antikörper oder ein humanisierter monoklonaler tierischer Antikörper ist.

4. Verwendung nach einem der Ansprüche 1 bis 3, bei der der zu verwendende blockierende Antikörper ausgewählt ist aus einem oder mehreren der Antikörper, die am 19.2.1999 unter den folgenden Bezeichnungen bei der DSMZ, Braunschweig, nach dem Budapester Vertrag hinterlegten Hybridomzellen produziert werden: (4E9/B2/C1) DSM ACC2389; (4C1/E1/G8) DSM ACC2390 ; (1B1/E10/B11/C12) DSM ACC2391 ; (7E3/F8/E3) DSM ACC2392 ; (3H10/A11/A1) DSM ACC2393.

5. Monoklonaler Antikörper gegen den hTSH-Rezeptor mit die Bindung von TSH und/oder von stimulierenden Autoantikörpern an den hTSH-Rezeptor blockierenden Eigenschaften, der **dadurch gekennzeichnet ist, daß** er eine kurze Peptidsequenz erkennt, die die Aminosäuren FDSH (Phe Asp Ser His) der Positionen 381-384 des hTSH-Rezeptors sowie gegebenenfalls zusätzliche flankierende Aminosäuren aus dessen extrazellulärer Domäne aufweist, und seine spezifische, antigenbindende Bindungseigenschaften aufweisenden Fragmente oder Konjugate.

6. Monoklonaler Antikörper nach Anspruch 5, **dadurch gekennzeichnet, daß** er ein humanisierter monoklonaler Antikörper aus der Maus ist.

7. Monoklonaler Antikörper, der von einer der unter den Bezeichnungen (4E9/B2/C1) DSM ACC2389; (4C1/E1/G8) DSM ACC2390; (1B1/E10/B11/C12) (DSM ACC2391; (7E3/F8/E3) DSM ACC2392; (3H10/A11/A1) DSM ACC2393 bei DSMZ, Braunschweig, nach dem Budapester Vertrag am 19.2.1999 hinterlegten Hybridomzellen produziert wird.

## Claims

1. Use of blocking antibodies against the human TSH receptor (hTSH receptor) which recognize a short peptide sequence comprising the amino acids FDSH (Phe Asp Ser His) of positions 381-384 of the hTSH receptor and optionally additional flanking amino acids from its extracellular domain, or of their specifically binding fragments, for the preparation of medicaments for the treatment of hyperthyroidism which is attributable to stimulating autoantibodies against the hTSH receptor (Graves' disease) or overstimulation of the thyroid gland by endogenous TSH or to activating mutations of the hTSH receptor, and of medicaments for the treatment of Graves' ophthalmopathy.

2. Use according to claim 1 for the preparation of medicaments to be administered by injection of infusion.

3. Use according to any of claims 1 or 3 wherein the blocking antibody to be used is a monoclonal human or animal antibody or a humanized monoclonal animal antibody.

4. Use according to any of claims 1 to 3 wherein the blocking antibody to be used is selected from one or more of the antibodies which are produced by hybridoma cells deposited on 19.02.1999 under the Budapest Treaty at DSMZ, Braunschweig under the following designations DSM ACC2389 (4E9/B2/C1); DSM ACC2390 (4C1/E1/G8); DSM ACC2391 (1B1/E10/B11/C12); DSM ACC2392 (7E3/F8/E3); DSM ACC2393 (3H10/A11/A1).

5. Monoclonal antibody against the hTSH receptor having properties which block the binding of TSH and/or stimulating autoantibodies with the hTSH receptor, which is **characterized in that** it recognizes a short peptide sequence comprising the amino acids FDSH (Phe Asp Ser His) of positions 381-384 of the hTSH receptor and optionally additional flanking amino acids from its extracellular domain, and fragments or conjugates having its specific antigen-binding properties.

6. Monoclonal antibody according to claim 5, **characterized in that** it is a humanized monoclonal mouse antibody.

7. Monoclonal antibody produced by one of the hybridoma cells deposited on 19.02.1999 under the Budapest Treaty at DSMZ, Braunschweig under the designations DSM ACC2389 (4E9/B2/C1); DSM ACC2390 (4C1/E1/G8); DSM ACC2391 (1B1/E10/B11/C12); DSM ACC2392 (7E3/F8/E3); DSM ACC2393 (3H10/A11/A1).

## Revendications

1. Utilisation d'anticorps bloquants dirigés contre le récepteur humain de la TSH (récepteur hTSH) qui reconnaissent une courte séquence de peptide qui présente les aminoacides FDSH (Phe Asp Ser His) dans les positions 381 - 384 du récepteur de la hTSH ainsi que, le cas échéant, des aminoacides supplémentaires concomitants de leur domaine extracellulaire ou de leurs fragments de liaison spécifiques, pour la fabrication de médicaments pour le traitement d'hyperthyréoses qui sont dues à des autoanti-corps stimulants dirigés contre le récepteur de la hTSH (Morbus Basedow) ou à une hyperstimulation de la glande thyroïdienne par une TSH endogène ou à des mutations activantes du récepteur de hTSH, et de médicaments pour le traitement de l'orbitopathie endocrine.

2. Utilisation selon la revendication 1 pour la fabrication de médicaments à administrer par injection ou infusion.

3. Utilisation selon la revendication 1 ou 2 dans le cadre de laquelle l'anticorps bloquant à utiliser est un anticorps monoclonal, humain ou animal, ou un anticorps monoclonal, animal humanisé.

4. Utilisation selon une des revendications 1 à 3, dans le cadre de laquelle on choisit comme anticorps bloquants à utiliser, un ou plusieurs des anticorps qui ont été produits par les cellules hybridomes déposées selon le Traité de Budapest, le 19.2.1999, auprès de la collection de cultures de cellules DSMZ, Braunschweig, sous les désignations suivantes: (4E9/B2/C1) DSM ACC2389; (4C1/E1/G8) DSM ACC2390; (1B1/E10/B11/C12) DSM ACC2391; (7E3/F8/E3) DSM ACC2392; (3H10/A11/A1) DSM ACC2393).

5. Anticorps monoclonal dirigé contre le récepteur de la hTSH avec des propriétés qui bloquent la liaison de TSH et /ou d'autoanticorps stimulants au récepteur de hTSH, **caractérisé en ce qu'**il reconnaît une courte séquence de peptide qui présente les aminoacides FDSH (Phe Asp Ser His) des positions 381 - 384 du récepteur de hTSH ainsi que, le cas échéant, des aminoacides supplémentaires concomitants de leur domaine extracellulaire, et leurs fragments ou conjugués présentant des propriétés de liaison antigéniques.

6. Anticorps monoclonal selon la revendication 5, **caractérisé en ce qu'**un anticorps monoclonal, humanisé provient de la souris.

7. Anticorps monoclonal qui est produit par l'une des cellules hybridomes déposée selon le Traité de Budapest, le 19.2.1999, auprès de la collection de cultures de cellules DSMZ, Braunschweig, sous les désignations (4E9/B2/C1) DSM ACC2389; (4C1/E1/G8) DSM ACC2390; (1B1/E10/B11/C12) DSM ACC2391; (7E3/F8/E3) DSM ACC2392; (3H10/A11/A1) DSM ACC2393).
